# EUROPÄISCHE PATENTSCHRIFT

(11) **EP 0 998 950 B1**
(45) Veröffentlichungstag und Bekanntmachung des Hinweises auf die Patenterteilung: **11.05.2005**
(21) Anmeldenummer: 99120529.5
(22) Anmeldetag: 15.10.1999
(51) Int. Cl.: A61M 1/36, A61M 1/16

(54) **Herz-Lungen-Maschine**
Cardio pulmonary system
Système cardiopulmonaire

(30) Priorität: 05.11.1998 DE 19851005
(43) Veröffentlichungstag der Anmeldung: 10.05.2000
(73) Patentinhaber: Jostra AG, 72145 Hirrlingen (DE)
(72) Erfinder: Heinze, Werner, Dipl.-Ing., 86923 Finning (DE)
(74) Vertreter: Möbus, Daniela, Dr.-Ing.

(56) Entgegenhaltungen:
- GB-A- 1 471 609
- US-A- 4 795 429
- US-A- 4 999 885
- US-A- 5 000 407
- US-A- 5 423 750
- US-A- 5 429 058

## Beschreibung

Die Erfindung betrifft eine Herz-Lungen-Maschine mit mindestens einer Pumpe für Blut oder andere Flüssigkeiten, die über Schlauchleitungen mit dem Körper eines Patienten verbindbar ist.

Eine derartige Herz-Lungen-Maschine ist durch die US 5,429,058 bekannt geworden.

Bei einer Operation sind Herz-Lungen-Maschinen über mehrere Schläuche mit dem Patienten verbunden. Abhängig von der Anzahl der Pumpen der Herz-Lungen-Maschine, die dabei im Einsatz sind, entsteht ein mehr oder weniger unübersichtliches Gewirr von Schläuchen. Tritt nun während der Operation ein für den Patienten kritischer Fall ein, so muss der Kardiotechniker unter Umständen mühsam und zeitraubend feststellen, welche Versorgungsleitung mit welcher Pumpe verbunden ist. Benötigt er dafür zu lange oder täuscht er sich bei der Lokalisierung des richtigen Schlauches, so kann dies für das Leben des Patienten sehr verhängnisvoll sein. Da die Schlauchleitungen lang genug sind, um knicken zu können, besteht hierin eine weitere Gefahr für das Leben des Patienten.

Durch die US 4,795,429 ist eine Schlauchverbindungsschnittstelle bekannt geworden.

Die Erfindung hat die Aufgabe, eine Herz-Lungen-Maschine dahin gehend zu verbessern, dass die oben genannten Gefahren der Maschinen weitestgehend reduziert werden.

Die Erfindung löst die gestellte Aufgabe durch eine Herz-Lungen-Maschine, die eine Schlauchverbindungsschnittstelle mit Aufnahmevorrichtungen für Schlauchleitungen aufweist, die mittels Schlauchleitungen mit der mindestens einen Pumpe verbindbar ist und an die auch zum Patienten führende Schlauchleitungen anschließbar sind, wobei an der Schlauchverbindungsschnittstelle Messgeräte zur Kontrolle der umgewälzten Flüssigkeiten angeordnet sind. Bei Einsatz mehrerer Pumpen gewähren die Aufnahmevorrichtungen für die Schlauchleitungen an der Schlauchverbindungsschnittstelle ein sicheres und verwechslungsfreies Nachvollziehen, welche der kurzen Schlauchleitungen mit welcher Pumpe verbunden ist. Außerdem lässt sich von der Schnittstelle aus auch leicht nachvollziehen, welche Schlauchleitung zu welchem Anschluss des Patienten führt. Durch die Schlauchverbindungsschnittstelle sind die Längen der einzelnen Leitungen verkürzt, besonders die Längen der Leitungen zwischen Schnittstelle und Pumpe, wodurch die Gefahr eines Abknickens der Leitungen stark vermindert wird. Durch die zentrale Anordnung der Messgeräte an einem bestimmten Ort weiß der Bediener sofort, ohne lange suchen zu müssen, wo er den jeweiligen Messwert ablesen muss.

Eine Auflagefläche der Schlauchverbindungsschnittstelle, auf der die pumpenseitigen und/oder die patientenseitigen Schlauchleitungen lösbar befestigt sind, kann das unübersichtliche Durcheinander und eine Verwechslung der Schlauchleitungen verhindern. Um die Schlauchleitungen ohne Verwechslungen anschließen zu können, können diese mindestens im Anschlussbereich farbig gekennzeichnet sein.

Die Schnittstelle kann an einem senkrechten Träger der Herz-Lungen-Maschine befestigt sein. Auf diese Weise kann die Schlauchverbindungsschnittstelle für das Bedienpersonal in einem ergonomischen Bedienungsbereich angeordnet werden. Aus demselben Grund kann auch die mindestens eine Pumpe sowie ein Bedientableau an dem senkrechten Träger der Herz-Lungen-Maschine angebracht sein.

Nachfolgend wird ein Ausführungsbeispiel einer erfindungsgemäß ausgebildeten Herz-Lungen-Maschine anhand der beiliegenden Zeichnung näher erläutert.

Im Einzelnen zeigen:
- Fig. 1: eine perspektivische Ansicht einer Herz-Lungen-Maschine;
- Fig. 2: eine perspektivische Ansicht einer Schlauchverbindungsschnittstelle;
- Fig. 3: eine perspektivische Ansicht einer an die Patientenseite angeschlossenen Herz-Lungen-Maschine.

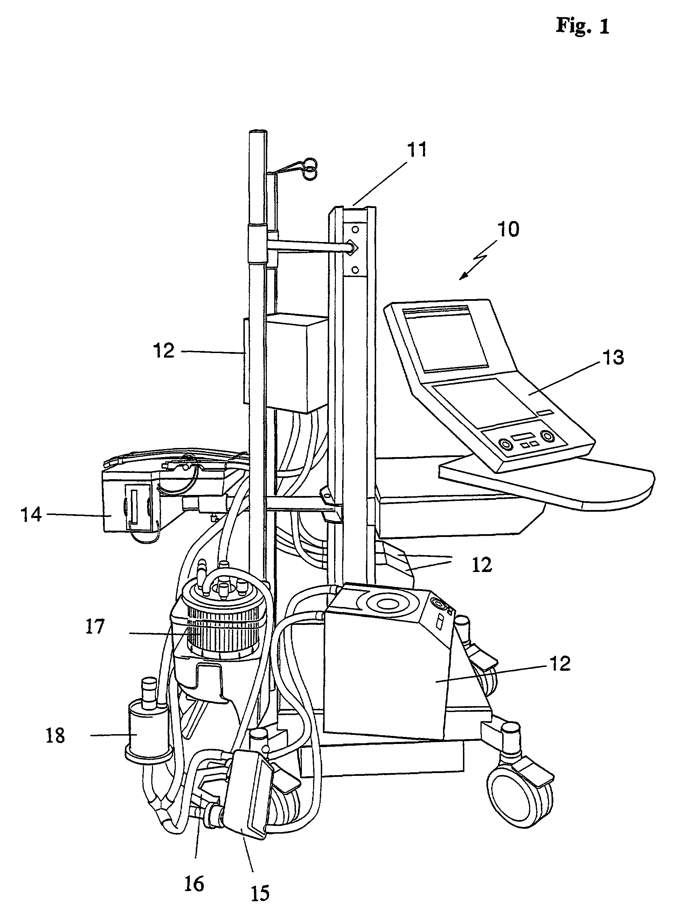

Fig. 1 zeigt eine Herz-Lungen-Maschine 10. In der Mitte der Herz-Lungen-Maschine 10 befindet sich ein zentral angeordneter senkrecht stehender Turm 11, an dem mehrere Komponenten der Herz-Lungen-Maschine 10 montiert sind, wie beispielsweise die Pumpen 12, ein Bedientableau 13, ein Oxygenator 15, der an einem Halter 16 befestigt ist, ein Reservoir 17, ein artieller Blutfilter 18 und eine Schlauchverbindungsschnittstelle 14. Die Pumpen 12 haben die Aufgabe, Blut oder andere Flüssigkeiten umzuwälzen. Mit dem Bedientableau 13 können die angeschlossenen Geräte der Herz-Lungen-Maschine 10 überwacht und bedient werden. Über die Schlauchverbindungsschnittstelle 14 sind die pumpenseitigen Schlauchleitungen mit den patientenseitigen Schlauchleitungen verbunden.

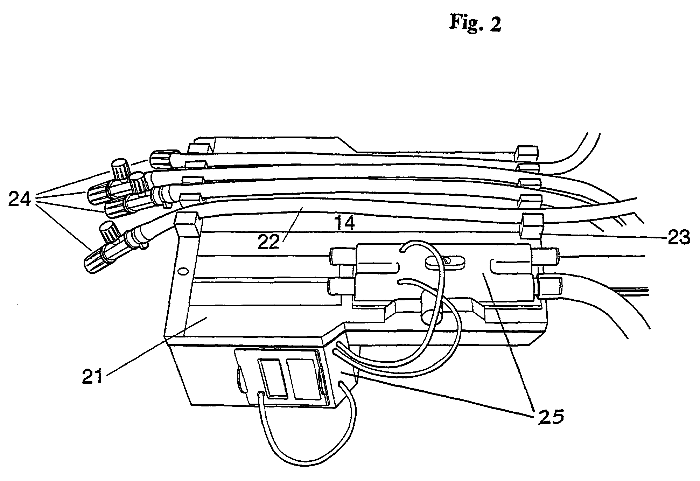

Fig. 2 zeigt die Schlauchverbindungsschnittstelle 14. Sie weist eine Auflagefläche 21 auf, auf der die pumpenseitigen und/oder die patientenseitigen Schlauchleitungen 22 mittels Schlauchklemmen 23 lösbar befestigt sind. Die Schlauchleitungen 22 sind farbig gekennzeichnet, beispielsweise mittels farbigen Verbindungselementen 24. Die Vorrichtungen 25 sind Messgeräte zur Kontrolle der umgewälzten Flüssigkeiten.

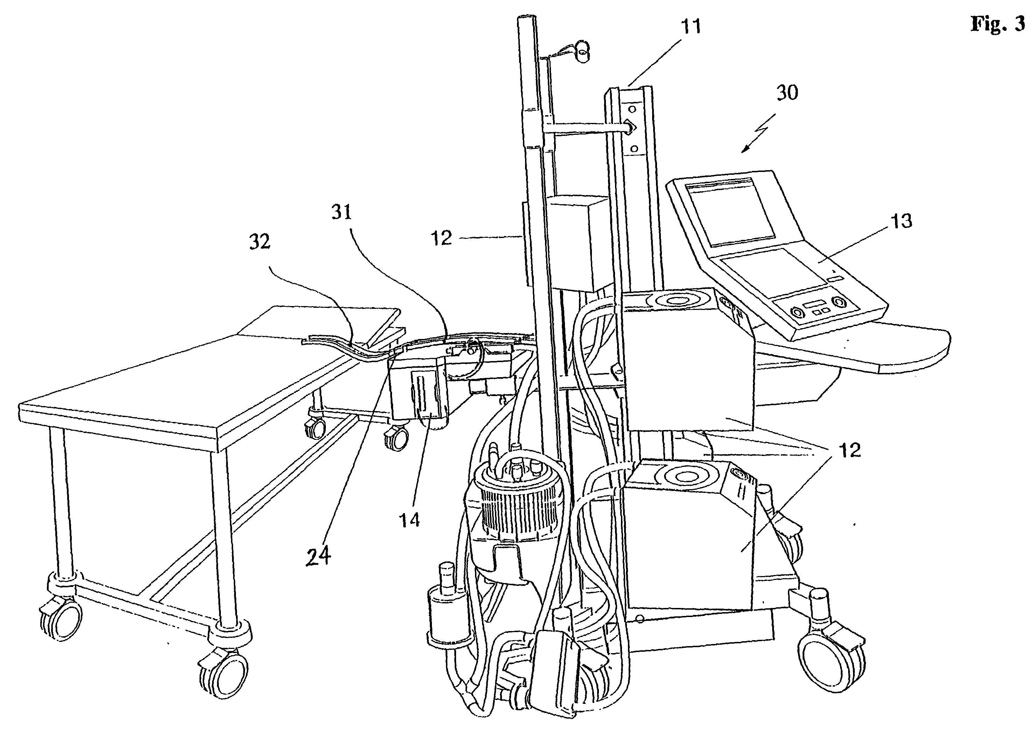

Fig. 3 zeigt eine Herz-Lungen-Maschine 30 mit Pumpen 12 für unterschiedliche Flüssigkeiten. An der vorzugsweise horizontal angeordneten Schlauchverbindungsschnittstelle 14 sind mittels der Verbindungselemente 24 pumpseitige Schlauchleitungen 31 mit patientenseitigen Schlauchleitungen 32 verbunden. Die patientenseitigen Schlauchleitungen 32 werden an den hier nicht näher dargestellten Patienten angeschlossen.

## Patentansprüche

1. Herz-Lungen-Maschine (10, 30) mit mindestens einer Pumpe (12) für Blut oder andere Flüssigkeiten, die über Schlauchleitungen (22) mit dem Körper eines Patienten verbindbar ist, **dadurch gekennzeichnet, dass** sie eine Schlauchverbindungsschnittstelle (14) mit Aufnahmevorrichtungen (23) für Schlauchleitungen (22) aufweist, die mittels Schlauchleitungen mit der mindestens einen Pumpe (12) verbindbar ist und an die zum Patienten führende Schlauchleitungen (22) anschließbar sand, und dass an der Schlauchverbindungsschnittstelle (14) Messgeräte (25) zur Kontrolle der umgewälzten Flüssigkeiten angeordnet sind.

2. Herz-Lungen-Maschine (10, 30) nach Anspruch 1, **dadurch gekennzeichnet, dass** die Schlauchverbindungsschnittstelle (14) eine Auflagefläche (21) aufweist, auf der die pumpenseitigen und/oder die patientenseitigen Schlauchleitungen (22) lösbar befestigt sind.

3. Herz-Lungen-Maschine (10, 30) nach Anspruch 1 oder 2, **dadurch gekennzeichnet, dass** die Schlauchleitungen (22) der Schlauchverbindungsschnittstelle (14) farbig gekennzeichnet sind.

4. Herz-Lungen-Maschine (10, 30) nach einem der Ansprüche 1 bis 3, **dadurch gekennzeichnet, dass** die Schlauchverbindungsschnittstelle (14) mit Farbmarkierungen versehen ist, die zu den Farbmarkierungen der Schlauchleitungen (22) passen.

5. Herz-Lungen-Maschine (10, 30) nach einem der Ansprüche 1 bis 4, **dadurch gekennzeichnet, dass** die Schlauchverbindungsschnittstelle (14) an einem senkrechten Träger (11) der Herz-Lungen-Maschine (10, 30) befestigt ist.

6. Herz-Lungen-Maschine (10, 30) nach Anspruch 5, **dadurch gekennzeichnet, dass** die mindestens eine Pumpe (12) an dem senkrechten Träger (11) der Herz-Lungen-Maschine (10, 30) angebracht ist.

## Claims

1. Cardio-pulmonary system (10, 30) with at least one pump (12) for blood or other fluids, which system is connectable by hose pipes (22) to the body of a patient, **characterised in that** it exhibits a hose connection interface (14) with receiving devices (23) for hose pipes (22), which interface is connectable by hose pipes to at least one pump (12) and to which hose pipes (22) leading to the patient are connectable, and **in that** measuring instruments (25) are arranged at the hose connection interface (14) for monitoring the circulated fluids.

2. Cardio-pulmonary system (10, 30) according to Claim 1, **characterised in that** the hose connection interface (14) exhibits a contact surface (21) on which the hose pipes (22) on the pump side and/or on the patient side are detachably secured.

3. Cardio-pulmonary system (10, 30) according to Claim 1 or 2, **characterised in that** the hose pipes (22) of the hose connection interface (14) are identified by colour.

4. Cardio-pulmonary system (10, 30) according to one of Claims 1 to 3, **characterised in that** the hose connection interface (14) is provided with colour markings which match the colour markings of the hose pipes (22).

5. Cardio-pulmonary system (10, 30) according to one of Claims 1 to 4, **characterised in that** the hose connection interface (14) is secured to a vertical support (11) of the cardio-pulmonary system (10, 30).

6. Cardio-pulmonary system (10, 30) according to Claim 5, **characterised in that** at least one pump (12) is installed on the vertical support (11) of the cardio-pulmonary system (10, 30).

## Revendications

1. Système cardiopulmonaire (10, 30) avec au moins une pompe (12) pour le sang ou d'autres liquides, qui peut être reliée par des conduites à tuyau (22) au corps d'un patient, **caractérisé en ce qu'**il comporte une interface de raccord pour tuyaux (14) avec des dispositifs de réception (23) pour des conduites à tuyau (22) qui peut être reliée à au moins une pompe (12) par des conduites à tuyau et à laquelle peuvent être raccordées les conduites à tuyau (22) menant au patient, et **en ce que** sur l'interface de raccord pour tuyaux (14) sont disposés des appareils de mesure (25) pour le contrôle des liquides en circulation.

2. Système cardiopulmonaire (10, 30) selon la revendication 1, **caractérisé en ce que** l'interface de raccord pour tuyaux (14) présente une surface de support (21) sur laquelle les conduites à tuyau (22) côté pompe et/ou côté patient sont fixées de manière séparable.

3. Système cardiopulmonaire (10, 30) selon la revendication 1 ou 2, **caractérisé en ce que** les conduites à tuyau (22) de l'interface de raccord pour tuyaux (14) sont identifiées par des couleurs.

4. Système cardiopulmonaire (10, 30) selon l'une des revendications 1 à 30, **caractérisé en ce que** l'interface de raccord pour tuyaux (14) est pourvue de marquages colorés qui sont adaptés aux marquages colorés des conduites à tuyau (22).

5. Système cardiopulmonaire (10, 30) selon l'une des revendications 1 à 4, **caractérisé en ce que** l'interface de raccord pour tuyaux (14) est fixée sur un support vertical (11) du système cardiopulmonaire (10, 30).

6. Système cardiopulmonaire (10, 30) selon la revendication 5, **caractérisé en ce que** ladite au moins une pompe (12) est fixée au support vertical (11) du système cardiopulmonaire (10, 30).
